# EUROPÄISCHE PATENTSCHRIFT

(11) **EP 0 757 042 B1**
(45) Veröffentlichungstag und Bekanntmachung des Hinweises auf die Patenterteilung: **10.09.2003**
(21) Anmeldenummer: 96115410.1
(22) Anmeldetag: 12.02.1994
(51) Int. Cl.: C07D 277/34, C07D 277/36, C07D 263/38, C07D 231/22, C07D 249/12, C07D 271/113, C07D 285/125, C07D 271/07, C07D 249/04, C07D 231/18, C07D 263/40, C07D 263/46, C07D 261/10, C07D 261/12, C07D 333/20

(54) **Ortho-substituierte 2-Methoxyiminophenylessigsäuremethylester**
Ortho-substituted 2-methoxyiminophenylaceticacid methylester
Ester méthylique de l'acide 2-méthoxyiminophénylacétique substitué en position ortho

(30) Priorität: 23.02.1993 DE 4305502
(43) Veröffentlichungstag der Anmeldung: 05.02.1997
(62) Teilanmeldung aus: 94907561.8
(73) Patentinhaber: BASF Aktiengesellschaft, 67063 Ludwigshafen (DE)
(72) Erfinder: Kirstgen, Reinhard, Dr., 67434 Neustadt (DE); Grammenos, Wassilios, Dr., 67063 Ludwigshafen (DE); Bayer, Herbert, Dr., 68159 Mannheim (DE); Doetzer, Reinhard, Dr., 69469 Weinheim (DE); Koenig, Hartmann, Dr., 67117 Limburgerhof (DE); Oberdorf, Klaus, Dr., 69117 Heidelberg (DE); Sauter, Hubert, Dr., 68167 Mannheim (DE); Wingert, Horst, Dr., 68159 Mannheim (DE); Lorenz, Gisela, Dr., 67434 Neustadt (DE); Ammermann, Eberhard, Dr., 64646 Heppenheim (DE); Harries, Volker, Dr., 67227 Frankenthal (DE)

(56) Entgegenhaltungen:
- EP-A- 0 299 694
- EP-A- 0 363 818
- EP-A- 0 378 308

## Beschreibung

Die vorliegende Erfindung betrifft Verbindungen der allgemeinen Formel II, 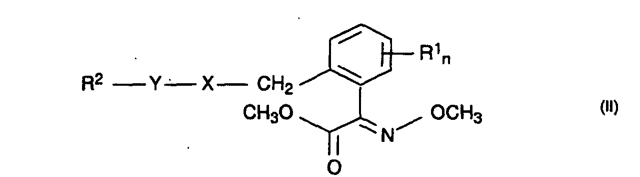 in der der Index und die Substituenten die folgende Bedeutung haben:
- n: 0, 1, 2, 3 oder 4, wobei die Reste R¹ verschieden sein können, wenn n größer als 1 ist;
- X: O oder S;
- R²⁻Y: für eine der folgenden Gruppen steht: 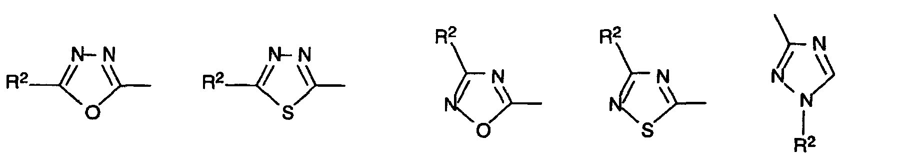 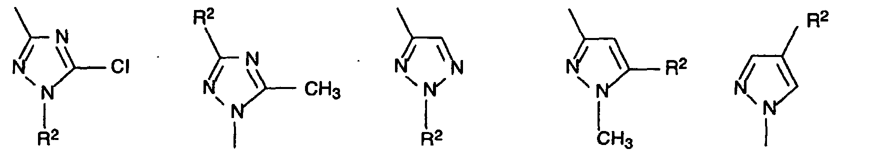 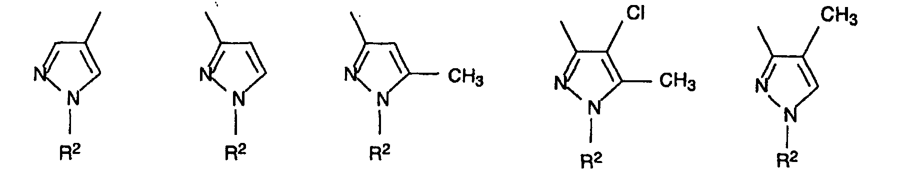 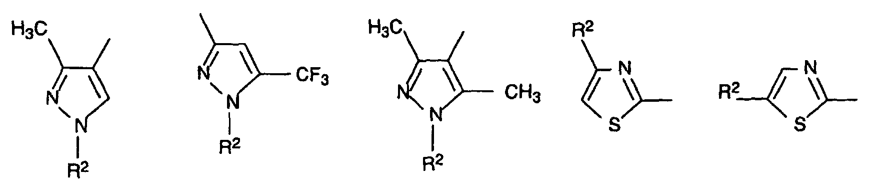 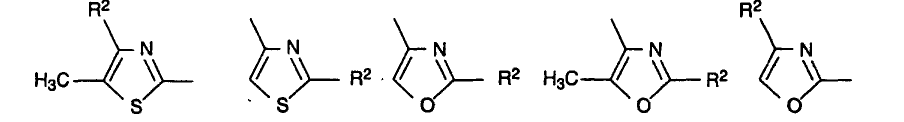 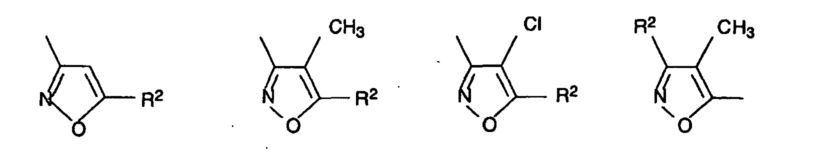
R¹ Nitro; Cyano; Halogen;
C₁-C₄-Alkyl; C₁-C₄-Halogenalkyl; C₁-C₄-Alkoxy; C₁-C₄-Halogen-alkoxy; C₁-C₄-Alkylthio;
Phenyl oder Phenoxy, wobei die aromatischen Ringe ein bis fünf Halogenatome oder einen bis drei der folgenden Reste tragen können: Halogen, C₁-C₄-Alkyl, C₁-C₄-Halogenalkyl, C₁-C₄-Alkoxy, C₁-C₄-Halogenalkoxy und C₁-C₄-Alkylthio;
oder, sofern n größer als 1 ist, eine an zwei benachbarte C-Atome des Phenylrestes gebundene 1,3-Butadien-1,4-diylgruppe, welche ihrerseits ein bis vier Halogenatome oder ein oder zwei der folgenden Reste tragen kann: Nitro, Cyano, Halogen, C₁-C₄-Alkyl, C₁-C₄-Halogenalkyl, C₁-C₄-Alkoxy, C₁-C₄-Halogenalkoxy und C₁-C₄-Alkylthio;
R² Wasserstoff;
C₁-C₆-Alkyl, C₂-C₆-Alkenyl, oder C₃-C₆-Alkinyl,
wobei die genannten Reste partiell oder vollständig halogeniert sein können und neben Halogenatomen zusätzlich ein bis drei der folgenden Subsitutenten tragen können:
Nitro, Cyano, Thiocyanato, C₁-C₄-Alkoxy, C₁-C₄-Halogenalkoxy, C₁-C₄-Alkylthio, C₁-C₄-Alkylamino, Di-C₁-C₄-alkylamino, C₃-C₆-Alkenyloxy, C₃-C₆-Alkinyloxy, C₁-C₆-Alkylcarbonyl, C₁-C₆-Alkoxyimino, C₃-C₆-Alkenyloxyimino, C₃-C₆-Alkinyloxyimino, C₁-C₆-Alkoxycarbonyl, C₁-C₆-Alkylthiocarbonyl, C₁-C₆-Alkylaminocarbonyl, Di-C₁-C₆-alkylaminocarbonyl, C₁-C₆-Alkylcarboxyl, C₁-C₆-Alkylcarbonylamino, C₃-C₇-Cycloalkyl, C₃-C₇-Cycloalkoxy, C₃-C₇-Cycloalkylthio, C₃-C₇-Cycloalkylamino, C₅-C₇-Cycloalkenyl,
5- bis 6-gliedrige, gesättigte oder ungesättigte Heterocyclen, enthaltend ein bis drei Stickstoffatome und/oder ein Sauerstoff- oder Schwefelatom,
Phenyl, 1-Naphthyl oder 2-Naphthyl,
5-Ring Heteroaromaten, enthaltend ein bis drei Stickstoffatome und/oder ein Sauerstoff- oder Schwefelatom,
6-Ring Heteroaromaten, enthaltend ein bis drei Stickstoffatome;
ein gesättigter oder ein- oder zweifach ungesättigter Ring, welcher neben Kohlenstoffatomen ein bis drei der folgenden Heteroatome als Ringglieder enthalten kann: Sauerstoff, Schwefel und Stickstoff,
wobei die genannten Ringe partiell oder vollständig halogeniert sein können und die ein- oder zweifach ungesättigten Ringe neben Halogenatomen zusätzlich ein bis drei der folgenden Substituenten tragen können:
Nitro, Cyano, C₁-C₆-Alkyl, C₁-C₄-Halogenalkyl, C₁-C₄-Alkoxy, C₁-C₄-Alkylthio, Di-C₁-C₄-alkylamino, C₂-C₆-Alkenyl, C₂-C₆-Alkinyl, C₁-C₆-Alkoxycarbonyl, C₁-C₆-Alkylaminocarbonyl, Di-C₁-C₆-alkylaminocarbonyl, C₁-C₆-Alkylcarboxyl, C₁-C₆-Alkylcarbonylamino, C₃-C₇-Cycloalkyl, Phenyl; oder ein ein- oder zweikerniger aromatischer Ring, welcher neben Kohlenstoffatomen ein bis vier Stickstoffatome oder ein oder zwei Stickstoffatome und ein Sauerstoff- oder schwefelatom oder ein Sauerstoff- oder Schwefelatom als Ringglieder enthalten kann,
wobei die genannten Ringe partiell oder vollständig halogeniert sein können, und neben Halogenatomen zusätzlich ein bis drei der folgenden Substituenten tragen können:
Nitro, Cyano, Thiocyanato, C₁-C₆-Alkyl, C₂-C₆-Alkenyl, C₃-C₆-Alkinyl, C₂-C₆-Alkinyloxy, C₁-C₆-Alkylcarbonyl, Di-C₁-C₆-alkylaminocarbonyl, C₁-C₆-Alkylcarboxyl, C₅-C₇-Cycloalkenyl,
5- bis 6-gliedrige, gesättigte oder ungesättigte Heterocyclen, enthaltend ein bis drei Stickstoffatome und/oder ein Sauerstoff- oder Schwefelatom,
Phenyl,
5-Ring Heteroaromaten enthaltend ein bis drei Stickstoffatome und/oder ein Sauerstoff- oder Schwefelatom,
6-Ring Heteroaromaten enthaltend ein bis drei Stickstoffatome.

Weiter betrifft die Erfindung die Verwendung dieser Verbindungen als Zwischenprodukte zur Herstellung von Verbindungen der Formel I, 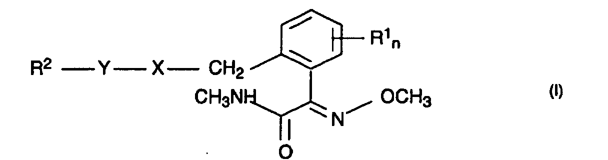 in der der Index und die Substituenten die in Anspruch 1 gegebene Bedeutung haben, durch Aminolyse.

Substituierte 2-Alkoxyiminophenylessigsäureamide sind bekannt (EP-A 398 692, EP-A 477 631, JP-A 4 182 461, WO 92/13 830 und GB 22 53 624). Die Verbindungen haben fungizide und z. T. auch insektizide Wirkung (EP-A 477 631). Ihre Wirkung ist jedoch unbefriedigend.

Überraschend wurde gefunden, daß die erfindungsgemäßen Verbindungen der allgemeinen Formel I eine stark verbesserte Wirksamkeit und Pflanzenverträglichkeit besitzen.

Die Herstellung der Verbindungen der Formel I erfolgt in Analogie zu verschiedenen aus der Literatur an sich bekannten Methoden.
Bei der Synthese der Verbindungen I ist die Reihenfolge, in der die Gruppierungen R²-Y-XCH₂- bzw. Gruppierung -C(=NOCH₃)-CO-NHCH₃ aus den entsprechenden Vorstufen aufgebaut werden, im allgemeinen unerheblich.

Besonders bevorzugt erhält man diese Gruppierungen nach den nachstehend beschriebenen Verfahren, wobei zur besseren Übersichtlichkeit in den folgenden Reaktionsschemata die jeweils nicht an der Umsetzung beteiligte Gruppe vereinfacht dargestellt ist:
die Gruppierung R²-Y-XCH₂- bzw. deren Vorstufe als R* und
die Gruppierung -C(=NOCH₃)-CO-NHCH₃ bzw. deren Vorstufe als R^{#}.

### A: Verfahren zur Synthese der Gruppierung R²-Y-XCH₂-

Man erhält die Verbindungen der allgemeinen Formeln II durch Umsetzung von IIA mit Hydroxy- bzw. Mercapto-substituierten fünfringheteroaromatischen Systemen III in inerten Lösungsmitteln in Gegenwart einer Base.

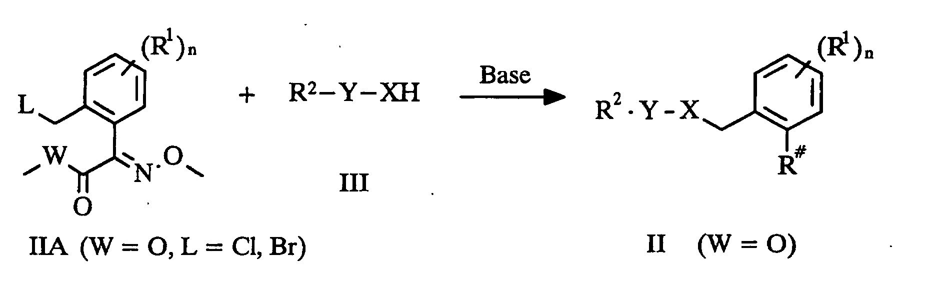

Mes = H₃CSO₂-

Tos = 4-CH₃-Phenyl-SO₂-

Diese Umsetzung wird üblicherweise bei Temperaturen von 0°C bis 80°C, vorzugsweise 20°C bis 60°C durchgeführt.

Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, Isopropanol, n-Butanol und tert.-Butanol, Ketone wie Aceton und Methylethylketon) sowie Dimethylsulfoxid, Dimethylformamid, Dimethylacetatamid, 1,3-Dimethylimidazolidin-2-on und 1,3-Dimethyltetrahydro-2(1H)-pyrimidinin besonders bevorzugt Methylenchlorid, Aceton und Dimethylformamid.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid, Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Kaliumcarbonate und Calziumcarbonat sowie Alkalimetallhydrogencarbonate wie Kaliumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkymagnesiumhalogenide wie Methylmagnesiumchlorid sowie Alkalimetall- und Erdalkalimetallalkoholate wie Natriummethanolat, Natriummethanolat, Kaliumethanolat, Kalium-tert.-butanolat und Dimethoxymagnesium außerdem organische Basen, z.B. tertiäre Amine wie Trimethylamin, Triethylamin, Di-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Natriumhydroxid, Natriumhydrid, Kaliumcarbonat und Kalium-tert.-butanolat.

Die Basen werden im allgemeinen äquimolar, im Überschuß oder gegebenenfalls als Lösungsmittel verwendet.

Es kann für die Umsetzung vorteilhaft sein, eine katalytische Menge eines Kronenethers wie z.B. 18-Krone-6 oder 15-Krone-5 zuzusetzen.

Die Umsetzung kann auch in Zweiphasensystemen bestehen aus einer Lösung von Alkali- oder Erdalkalihydroxiden oder Alkali- oder Erdalkalicarbonaten in Wasser und einer organischen Phase wie z.B. halogenierten Kohlenwasserstoffen durchgeführt werden. Als Phasentransferkatalysatoren können Ammoniumhalogenide und -tetrafluoroborate wie z.B. Benzyltriethylammoniumchlorid, Benzyltributylammoniumbromid, Tetrabutylammoniumchlorid, Hexadecyltrimethylammoniumbromid oder Tetrabutylammoniumtetrafluoroborat sowie Phosphoniumhalogenide wie Tetrabutylphosphoniumchlorid oder Tetraphenylphosphoniumbromid eingesetzt werden.

Es kann für die Umsetzung vorteilhaft sein, zunächst die Verbindungen III mit Base zu behandeln und das resultierende Salz mit der Verbindung IIA umzusetzen.

Die Darstellung der Ausgangsverbindungen IIA ist in der Literatur mehrfach beschrieben, vgl. EP-A 363 818.

Die Darstellung der Hydroxy- bzw. Mercapto-Fünfringheterocyclen III ist in der Literatur beschrieben, bzw. kann ananlog den dort gezeigten Synthesewegen durchgeführt werden, vgl. J. Heterocycl. Chem. 19 (1982), 541 ff; Acta Chem. Scand. 7 (1953), 374 ff; Chem. Pharm. Bull. 33 (1985), 3479 ff; Acta Chem. Scand. 17 (1963), 144 ff; Canadian Patent 1 177 081; Can. J. Chem. 57 (1979), 904 ff; Ann. Chem. 338 (1905), 273 ff; DOS1029827; Chem. Pharm. Bull. 15 (1967), 1025 ff; Agric. Biol. Chem. 50 (1986), 1831 ff; J. Org. Chem. 23 (1958), 1021 ff; Chem. Ber. 22 (1889), 2433 ff; Chem. Ber. 24 (1891), 369 ff; J. Med. Chem. 15 (1971), 39 ff; J. Am. Chem. Soc. 76 (1954), 4450 ff.

### B: Verfahren zur Synthese der Gruppierung -C(=NOCH₃)-CO-NHCH₃

Man erhält die Verbindungen der allgemeinen Formel I durch Aminolyse der entsprechenden 2-Methoxyimino-phenylessigsäureester II (vgl. Houben-Weyl Bd. E 5, S. 983 ff).

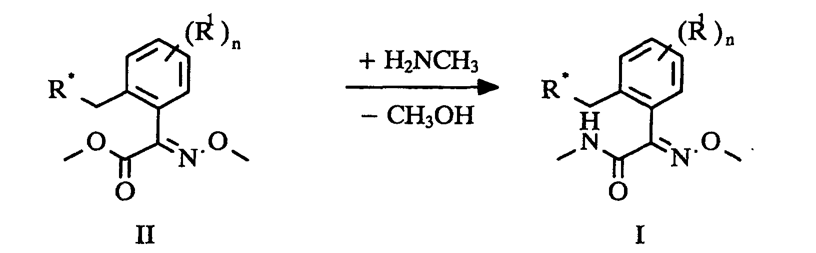

Diese Umsetzung wird üblicherweise bei Temperaturen von 0°C bis 60°C, vorzugsweise 10°C bis 30°C durchgeführt.

Methylamin kann durch gasförmiges Einleiten oder als wäßrige Lösung zu einer Lösung von II zudosiert werden.

Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Alkohole wie Methanol, Ethanol, n-Propanol, Isopropanol, n-Butanol und tert.-Butanol, besonders bevorzugt Methanol, Toluol und Tetrahydrofuran.

Es können auch Gemische der genannten Lösungsmittel verwendet werden.

Im Hinblick auf die biologische Wirkung der Verbindungen I gegen Schädlinge wie insbesondere Schadpilze, Insekten, Nematoden und Spinnentiere kommen insbesondere solche Verbindungen II in Betracht, in denen der Index und die Substituenten die folgende Bedeutung haben:

Von besonderem Interesse sind solche Verbindungen, in denen Y für 3-Pyrazolyl, 4-Thiazolyl und 1,2,4-Triazol-3-yl steht.

Des weiteren werden Verbindungen der Formel II bevorzugt, in denen
- n: für 0 oder 1 steht;
- X: für O oder S steht;
- Y: für ein fünfringheteroaromatisches System, das neben R² noch ein oder zwei Reste aus der Gruppe Cl, CH₃, CF₃ oder OCH₃ tragen kann, steht;
- R¹: für Halogen, C₁-C₄-Alkyl, C₁-C₂-Halogenalkyl, C₁-C₄-Alkoxy, C₁-C₂-Halogenalkoxy, C₁-C₂-Alkylthio oder Phenyl steht;
- R²: für ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann, steht.

Außerdem werden Verbindungen der Formel II bevorzugt, in denen
- n: für 0 steht;
- X: für O steht;
- Y: für ein fünfringheteroaromatisches System, das neben R² noch ein oder zwei Reste aus der Gruppe Cl, CH₃, CF₃ oder OCH₃ tragen kann, steht;
- R²: für ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann, steht.

Daneben werden Verbindungen der Formel II bevorzugt, in denen
- n: für 0 oder 1 steht;
- X: für O oder S steht;
- Y: für ein fünfringheteroaromatisches System, das neben R² noch ein oder zwei Reste aus der Gruppe Cl, CH₃, CF₃ oder OCH₃ tragen kann, steht;
- R¹: für Halogen, C₁-C₄-Alkyl, C₁-C₂-Halogenalkyl, C₁-C₄-Alkoxy, C₁-C₂-Halogenalkoxy, C₁-C₂-Alkylthio oder Phenyl steht;
- R²: für ggf. substituiertes Phenyl steht.

Des weiteren werden Verbindungen der Formel II bevorzugt, in denen
- n: für 0 steht;
- X: für O steht;
- Y: für ein fünfringheteroaromatisches System, das neben R² noch ein oder zwei Reste aus der Gruppe Cl, CH₃, CF₃ oder OCH₃ tragen kann, steht;
- R²: für ggf. substituiertes Phenyl steht.

Weiter werden Verbindungen der Formel II bevorzugt, in denen
- n: für 0 oder 1 steht;
- X: für O oder S steht;
- Y: für ein fünfringheteroaromatisches System, das neben R² noch ein oder zwei Reste aus der Gruppe Cl, CH₃, CF₃ oder OCH₃ tragen kann, steht;
- R¹: für Halogen, C₁-C₄-Alkyl, C₁-C₂-Halogenalkyl, C₁-C₄-Alkoxy, C₁-C₂-Halogenalkoxy, C₁-C₂-Alkylthio oder Phenyl steht;
- R²: für ggf. substituierten Fünfringheteroaromaten steht.

Außerdem werden Verbindungen der Formel II bevorzugt, in denen
- n: für 0 oder 1 steht;
- X: für O oder S steht;
- Y: für ein sechsringheteroaromatisches System, das neben R² noch ein oder zwei Reste aus der Gruppe Cl, CH₃, CF₃ oder OCH₃ tragen kann, steht;
- R²: für ggf. substituierten Sechsringheteroaromaten steht.

Die in den nachstehenden Synthesebeispielen wiedergegebenen Vorschriften wurden unter entsprechender Abwandlung der Ausgangsverbindungen zur Herstellung weiterer Verbindungen benutzt. Die so erhaltenen Verbindungen sind in der nachstehenden Tabelle mit physikalischen Angaben aufgeführt.

### Beispiel 1

### E-2-Methoxyimino-2-[(2-[5-(4-chlorphenyl)-isoxazol-3-yl]-oxymethyl)-phenyl]-essigsäuremethylester

1,8 g 3-Hydroxy-5-(4-chlorphenyl)-isoxazol werden in 10 ml Dimethylformamid gelöst. Hierzu gibt man 1,6 g fein gepulvertes Kaliumcarbonat und 2,8 g E-2-Methoxyimino-2-[(2-brommethyl)-phenyl]-essigsäuremethylester. Man rührt über Nacht bei Raumtemperatur und gießt dann zur Aufarbeitung den Reaktionsansatz auf 50 ml Wasser. Nach dreimaligem Extrahieren mit Methyltert.butylether werden die vereinigten organischen Phasen über Natriumsulfat getrocknet. Nach Abziehen des Lösungsmittels wird der Rückstand mit Methyl-tert.butylether verrieben. Nach Absaugen verbleiben 1,8 g der Titelverbindung.
Fp.: 155 - 157°C
¹H-NMR (CDCl₃, δ in ppm):
3,85 (s, 3H); 4,06 (s, 3H); 5,2 (s, 2H); 6,1 (s, 1H); 7,2-7,7 (m, 8H)

### Beispiel 2

### E-2-Methoxyimino-2-[(2-[5-(4-chlorphenyl)-isoxazol-3-yl]-oxymethyl)- phenyl]-essigsäuremethylamid

1,0 g des Methylesters aus Beispiel 1 werden in 15 ml Tetrahydrofuran gelöst und mit 1 ml 40 %iger wäßriger Methylaminlösung versetzt. Man rührt über Nacht bei Raumtemperatur, engt den Ansatz ein und nimmt den Rückstand in 50ml Methyl-tert.butylether auf. Die organische Phase wird mit Wasser extrahiert, über Natriumsulfat getrocknet und abschließend bis zur Trockne eingedampft. Es verbleiben 0,9 g der Titelverbindung als farbloser Feststoff.
Fp.: 195 - 198°C
¹H-NMR (CDCl₃, δ in ppm):
2,95 (d, 3H); 3,95 (s, 3H); 5,2 (s, 2H); 6,15 (s, 1H); 6,85 (NH); 7,2-7,8 (m, 8H)

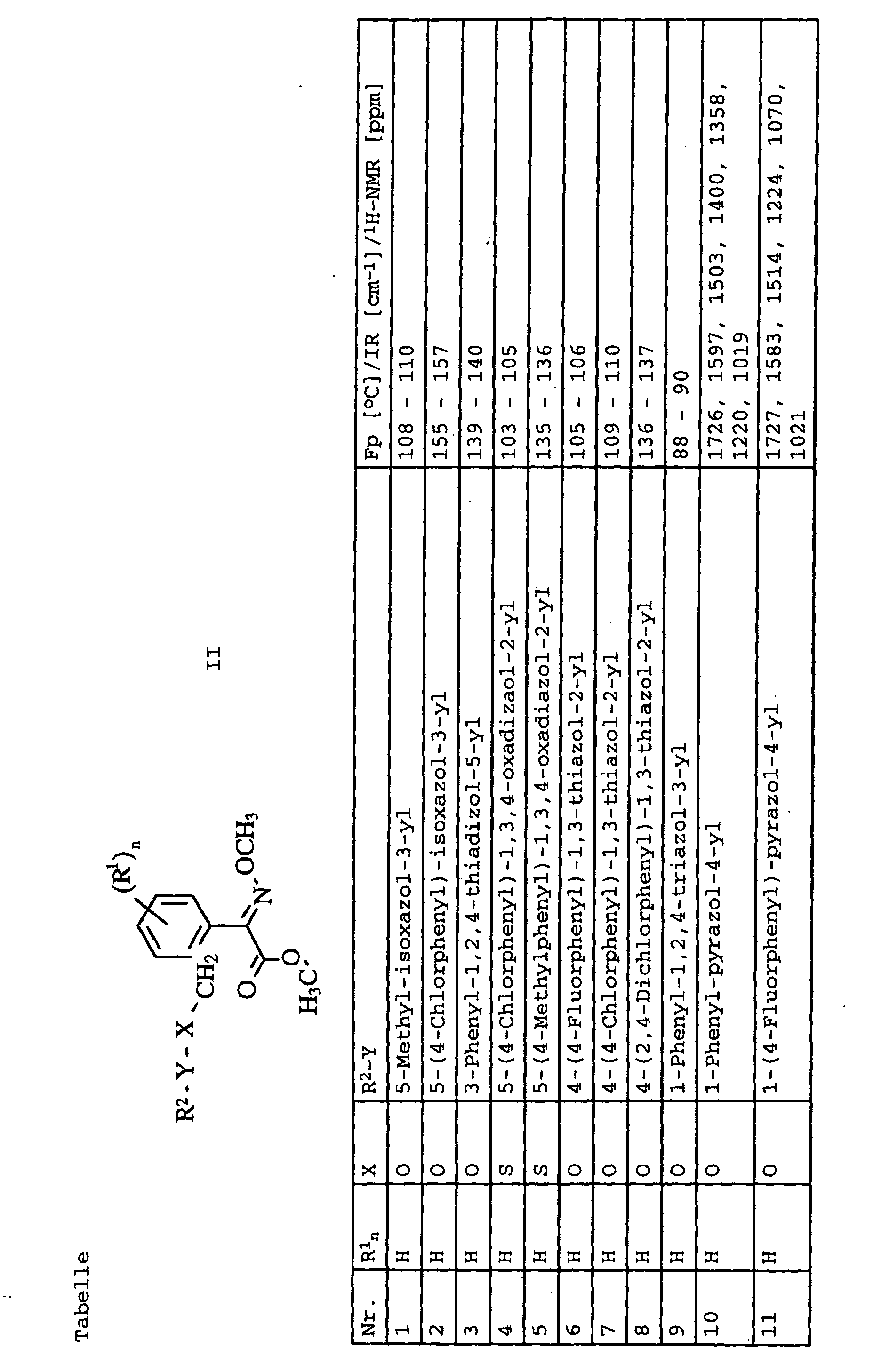

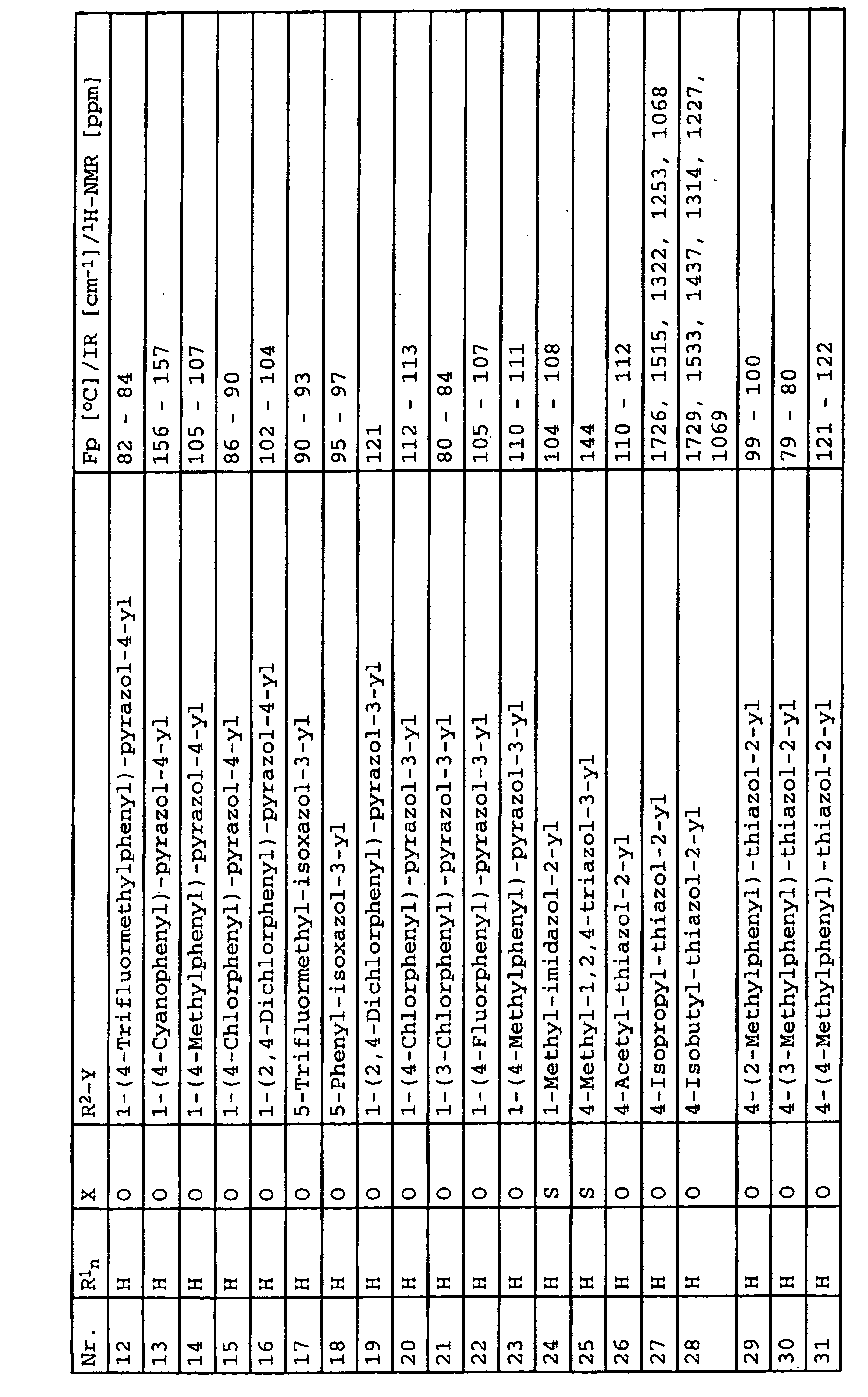

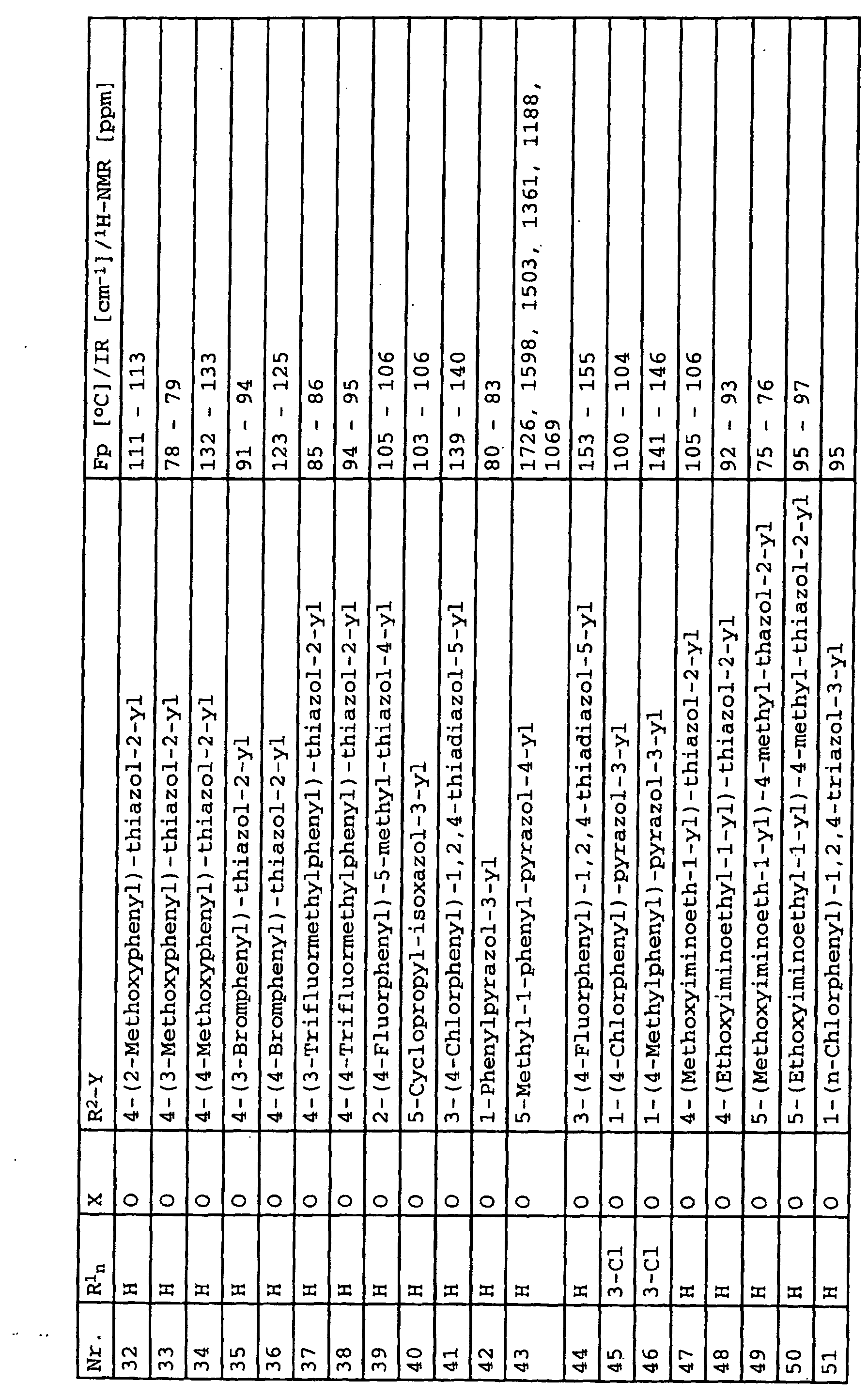

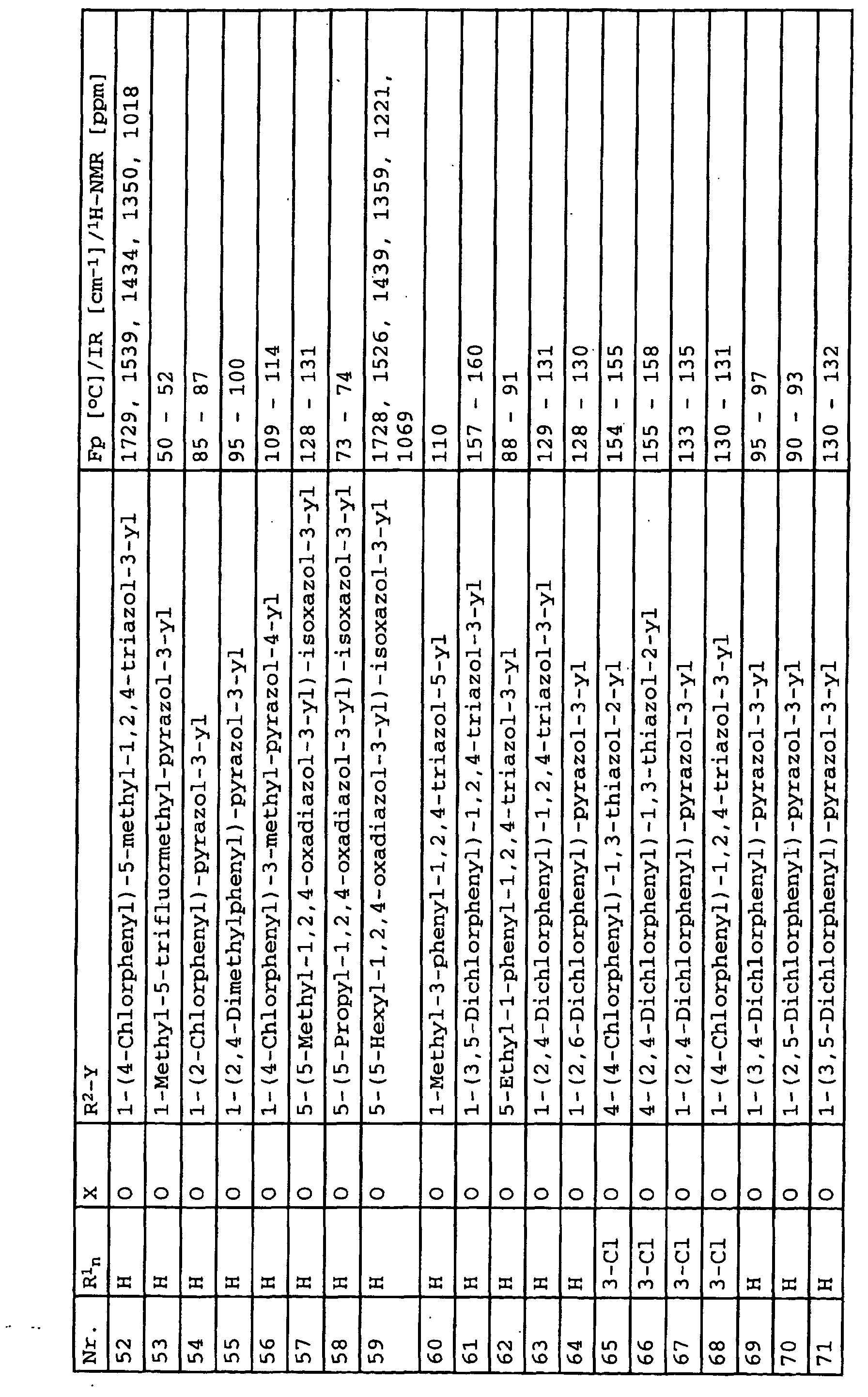

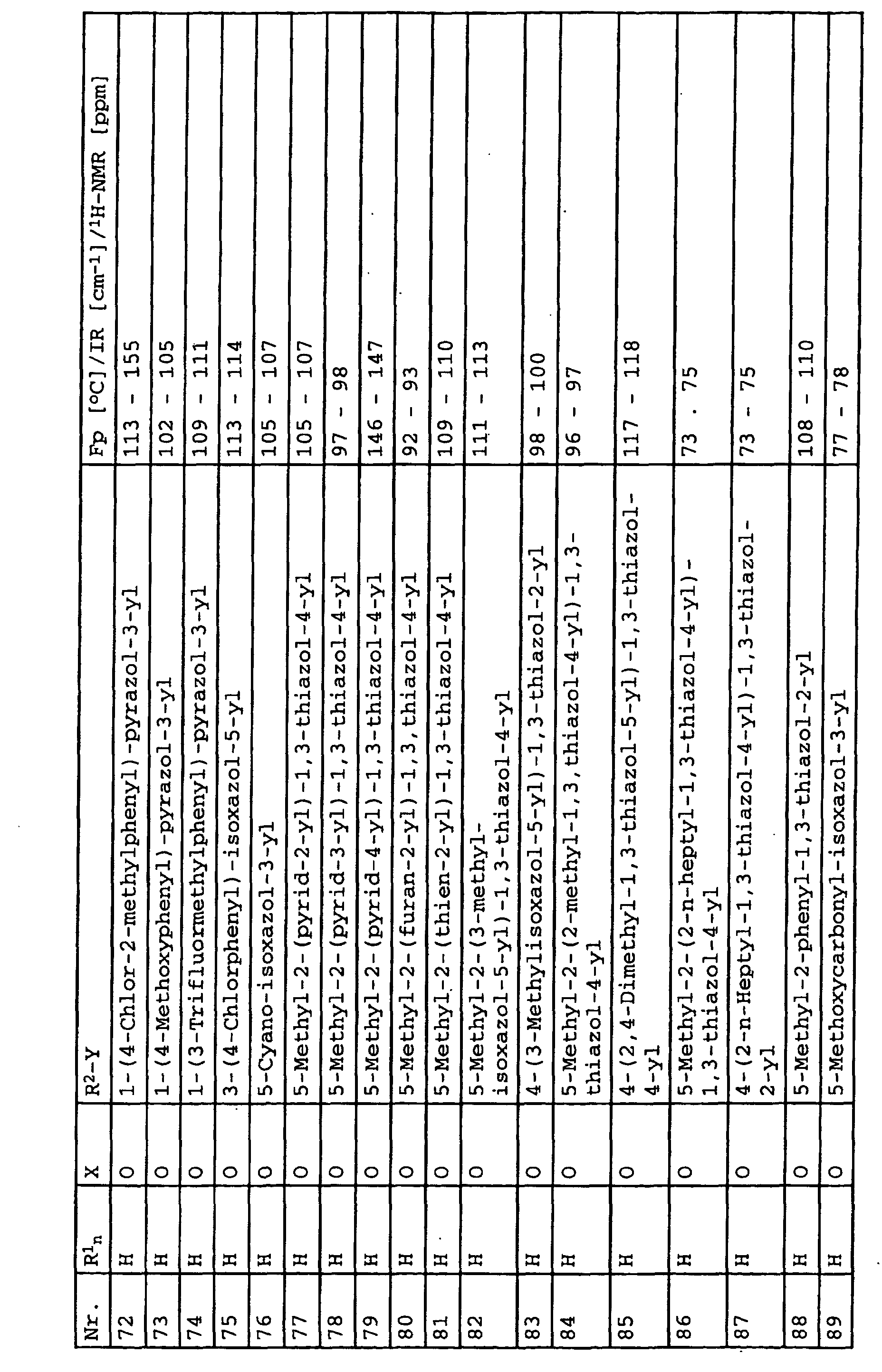

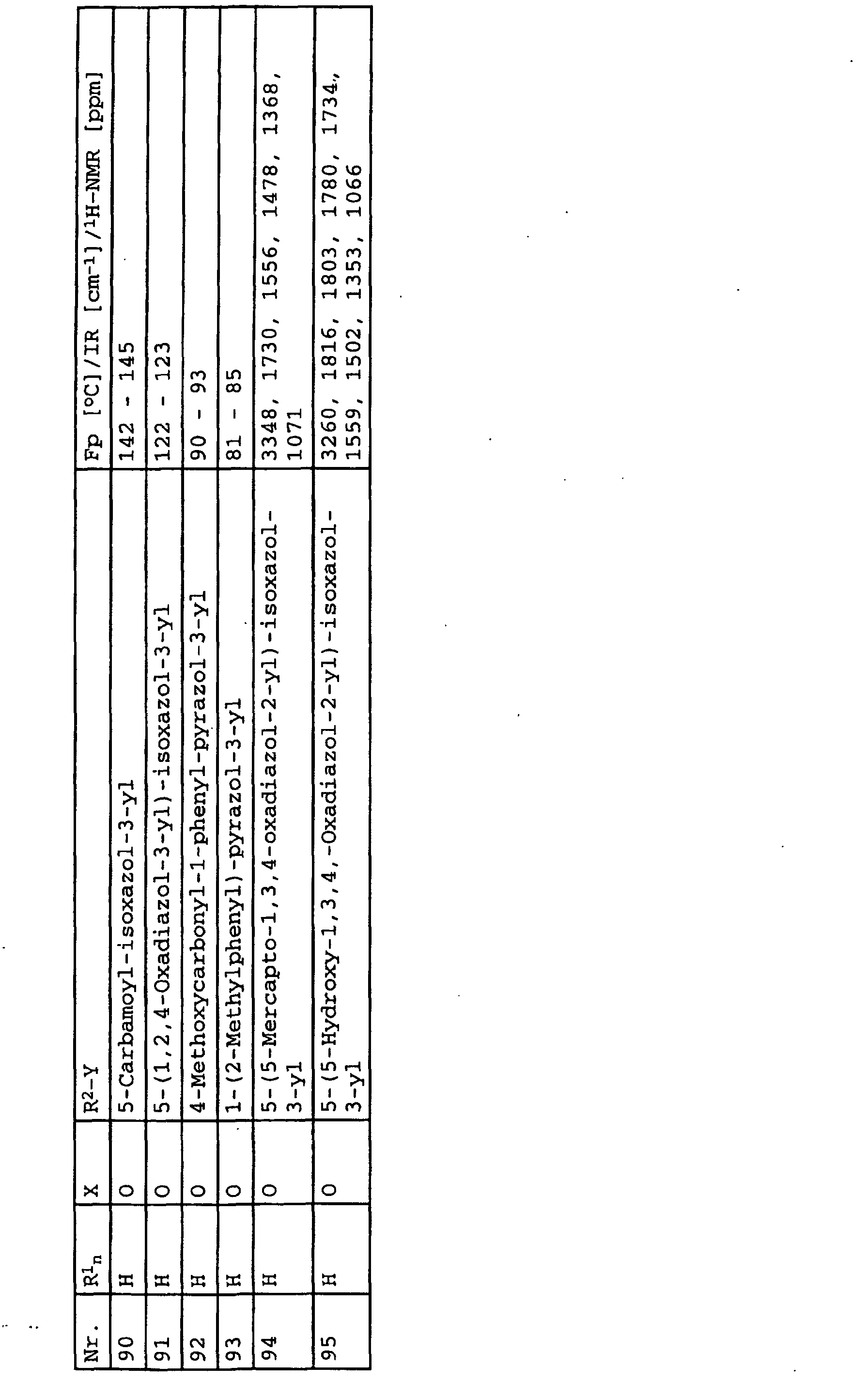

## Patentansprüche

1. Verbindungen der allgemeinen Formel II 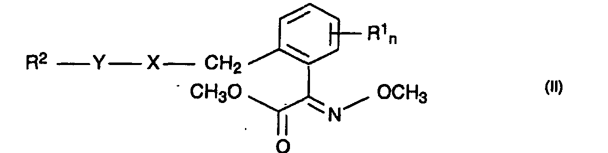 in der der Index und die Substituenten die folgende Bedeutung haben:
n 0, 1, 2, 3 oder 4, wobei die Reste R¹ verschieden sein können, wenn n größer als 1 ist;
X O oder S;
R²⁻Y für eine der folgenden Gruppen steht: 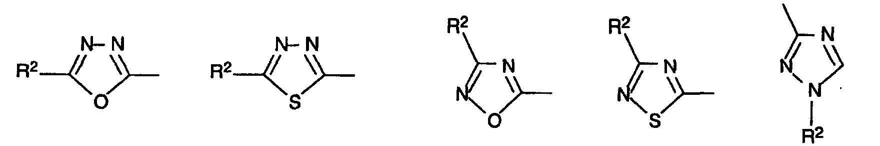 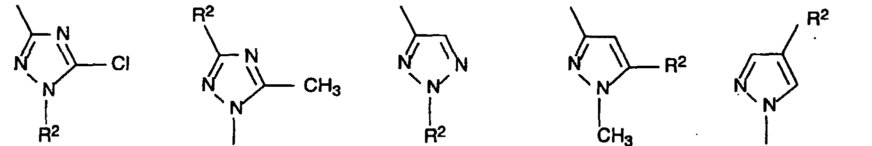 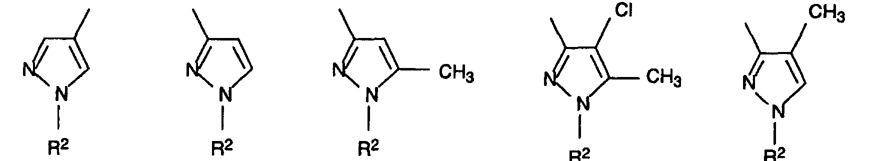 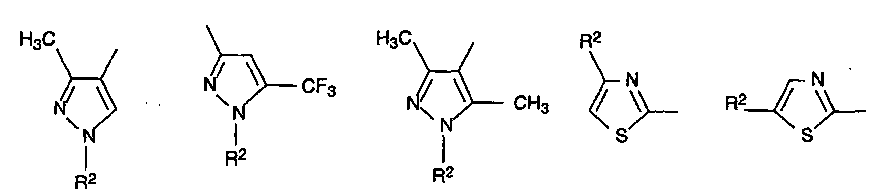 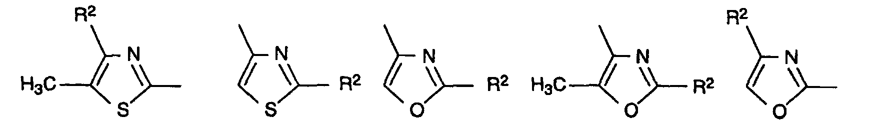 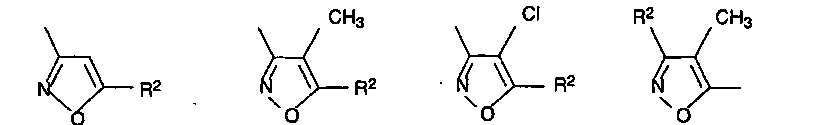
R¹ Nitro; Cyano; Halogen;
C₁-C₄-Alkyl; C₁-C₄-Halogenalkyl; C₁-C₄-Alkoxy; C₁-C₄-Halogenalkoxy; C₁-C₄-Alkylthio;
Phenyl oder Phenoxy, wobei die aromatischen Ringe ein bis fünf Halogenatome oder einen bis drei der folgenden Reste tragen können: Halogen, C₁-C₄-Alkyl, C₁-C₄-Halogenalkyl, C₁-C₄-Alkoxy, C₁-C₄-Halogenalkoxy und C₁-C₄-Alkylthio;
oder, sofern n größer als 1 ist, eine an zwei benachbarte C-Atome des Phenylrestes gebundene 1,3-Butadien-1,4-diylgruppe, welche ihrerseits ein bis vier Halogenatome oder ein oder zwei der folgenden Reste tragen kann: Nitro, Cyano, Halogen, C₁-C₄-Alkyl, C₁-C₄-Halogenalkyl, C₁-C₄-Alkoxy, C₁-C₄-Halogenalkoxy und C₁-C₄-Alkylthio;
R² Wasserstoff;
C₁-C₆-Alkyl, C₂-C₆-Alkenyl, oder C₃-C₆-Alkinyl,
wobei die genannten Reste partiell oder vollständig halogeniert sein können und neben Halogenatomen zusätzlich ein bis drei der folgenden Subsitutenten tragen können:
Nitro, Cyano, Thiocyanato, C₁-C₄-Alkoxy, C₁-C₄-Halogenalkoxy, C₁-C₄-Alkylthio, C₁-C₄-Alkylamino, Di-C₁-C₄-alkylamino, C₃-C₆-Alkenyloxy, C₃-C₆-Alkinyloxy, C₁-C₆-Alkylcarbonyl, C₁-C₆-Alkoxyimino, C₃-C₆-Alkenyloxyimino, C₃-C₆-Alkinyloxyimino, C₁-C₆-Alkoxycarbonyl, C₁-C₆-Alkylthiocarbonyl, C₁-C₆-Alkylaminocarbonyl, Di-C₁-C₆-alkylaminocarbonyl, C₁-C₆-Alkylcarboxyl, C₁-C₆-Alkylcarbonylamino, C₃-C₇-Cycloalkyl, C₃-C₇-Cycloalkoxy, C₃-C₇-Cycloalkylthio, C₃-C₇-Cycloalkylamino, C₅-C₇-Cycloalkenyl,
5- bis 6-gliedrige, gesättigte oder ungesättigte Heterocyclen, enthaltend ein bis drei Stickstoffatome und/oder ein Sauerstoff- oder Schwefelatom,
Phenyl, 1-Naphthyl oder 2-Naphthyl,
5-Ring Heteroaromaten, enthaltend ein bis drei Stickstoffatome und/oder ein Sauerstoff- oder Schwefelatom,
6-Ring Heteroaromaten, enthaltend ein bis drei Stickstoffatome;
ein gesättigter oder ein- oder zweifach ungesättigter Ring, welcher neben Kohlenstoffatomen ein bis drei der folgenden Heteroatome als Ringglieder enthalten kann: Sauerstoff, Schwefel und Stickstoff,
wobei die genannten Ringe partiell oder vollständig halogeniert sein können und die ein- oder zweifach ungesättigten Ringe neben Halogenatomen zusätzlich ein bis drei der folgenden Substituenten tragen können:
Nitro, Cyano, C₁-C₆-Alkyl, C₁-C₄-Halogenalkyl, C₁-C₄-Alkoxy, C₁-C₄-Alkylthio, Di-C₁-C₄-alkylamino, C₂-C₆-Alkenyl, C₂-C₆-Alkinyl, C₁-C₆-Alkoxycarbonyl, C₁-C₆-Alkylaminocarbonyl, Di-C₁-C₆-alkylaminocarbonyl, C₁-C₆-Alkylcarboxyl, C₁-C₆-Alkylcarbonylamino, C₃-C₇-Cycloalkyl, Phenyl;
oder ein ein- oder zweikerniger aromatischer Ring, welcher neben Kohlenstoffatomen ein bis vier Stickstoffatome oder ein oder zwei Stickstoffatome und ein Sauerstoff- oder schwefelatom oder ein Sauerstoff- oder Schwefelatom als Ringglieder enthalten kann,
wobei die genannten Ringe partiell oder vollständig halogeniert sein können, und neben Halogenatomen zusätzlich ein bis drei der folgenden Substituenten tragen können:
Nitro, Cyano, Thiocyanato, C₁-C₆-Alkyl, C₂-C₆-Alkenyl, C₂-C₆-Alkinyl, C₃-C₆-Alkinyloxy, C₁-C₆-Alkylcarbonyl, Di-C₁-C₆-alkylaminocarbonyl, C₁-C₆-Alkylcarboxyl, C₅-C₇-Cycloalkenyl,
5- bis 6-gliedrige, gesättigte oder ungesättigte Heterocyclen, enthaltend ein bis drei Stickstoffatome und/oder ein Sauerstoff- oder Schwefelatom,
Phenyl,
5-Ring Heteroaromaten enthaltend ein bis drei Stickstoffatome und/oder ein Sauerstoff- oder Schwefelatom,
6-Ring Heteroaromaten enthaltend ein bis drei Stickstoffatome.

2. Verbindungen der Formel II gemäß Anspruch 1, in denen Y für 3-Pyrazolyl, 4-Thiazolyl oder 1,2,4-Triazol-3-yl steht.

3. Verbindungen der Formel II gemäß Anspruch 1, in -denen der Index und die Substituenten die folgende Bedeutung haben:
n 0 oder 1;
X O oder S;
R¹ Halogen, C₁-C₄-Alkyl, C₁-C₂-Halogenalkyl, C₁-C₄-Alkoxy, C₁-C₂-Halogenalkoxy, C₁-C₂-Alkylthio oder Phenyl;
R² Phenyl, welches unsubstituiert oder partiell oder vollständig halogeniert sein kann und neben Halogenatomen zusätzlich ein bis drei der folgenden Substituenten tragen kann:
Nitro, Cyano, Thiocyanato, C₁-C₆-Alkyl, C₂-C₆-Alkenyl, C₃-C₆-Alkinyl, C₃-C₆-Alkinyloxy, C₁-C₆-Alkylcarbonyl, Di-C₁-C₆-alkylaminocarbonyl, C₁-C₆-Alkylcarboxyl, C₅-C₇-Cycloalkenyl,
5- bis 6-gliedrige, gesättigte oder ungesättigte Heterocyclen, enthaltend ein bis drei Stickstoffatome und/oder ein Sauerstoff- oder Schwefelatom,
Phenyl,
5-Ring Heteroaromaten enthaltend ein bis drei Stickstoffatome und/oder ein Sauerstoff- oder Schwefelatom,
6-Ring Heteroaromaten enthaltend ein bis drei Stickstoffatome.

4. Verbindungen der Formel II gemäß Anspruch 1, in denen der Index und die Substituenten die folgende Bedeutung haben:
n 0
X O;
R¹ Halogen, C₁-C₄-Alkyl, C₁-C₂-Halogenalkyl, C₁-C₄-Alkoxy, C₁-C₂-Halogenalkoxy, C₁-C₂-Alkylthio oder Phenyl;
R² Phenyl, unsubstituiert oder substituiert mit Halogen, Cyano, C₁-C₄-Alkyl, C₁-C₄-Alkoxy, C₁-C₂-Halogenalkyl, C₁-C₂-Halogenalkoxy, Phenyl oder Oxy-C₁-C₂-alkylidenoxy.

5. Verbindungen der Formel II.1 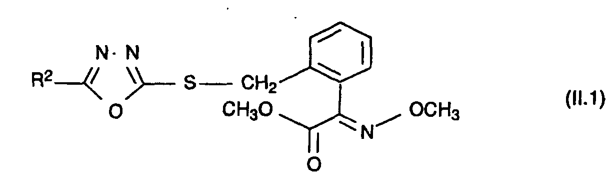 in denen R² für Phenyl steht, wobei Phenyl unsubstituiert oder substituiert mit Halogen, Cyano, C₁-C₄-Alkyl, C₁-C₄-Alkoxy, C₁-C₂-Halogenalkyl, C₁-C₂-Halogenalkoxy, Phenyl oder Oxy-C₁-C₂-alkylidenoxy ist.

6. Verbindungen der Formel II.2 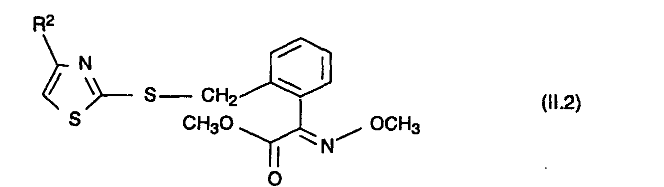 in denen R² für Phenyl steht, wobei Phenyl unsubstituiert oder substituiert mit Halogen, Cyano, C₁-C₄-Alkyl, C₁-C₄-Alkoxy, C₁-C₂-Halogenalkyl, C₁-C₂-Halogenalkoxy, Phenyl oder Oxy-C₁-C₂-alkylidenoxy ist.

7. Verbindungen der Formel II.3 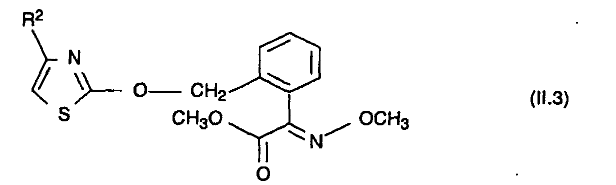 in denen R² für Phenyl steht, wobei Phenyl unsubstituiert oder substituiert mit Halogen, Cyano, C₁-C₄-Alkyl, C₁-C₄-Alkoxy, C₁-C₂-Halogenalkyl, C₁-C₂-Halogenalkoxy, Phenyl oder Oxy-C₁-C₂-alkylidenoxy ist.

8. Verbindungen der Formel II.5 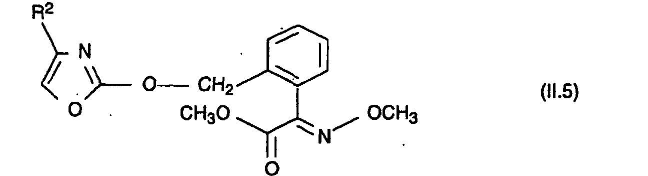 in denen R² für Phenyl steht, wobei Phenyl unsubstituiert oder substituiert mit Halogen, Cyano, C₁-C₄-Alkyl, C₁-C₄-Alkoxy, C₁-C₂-Halogenalkyl, C₁-C₂-Halogenalkoxy, Phenyl oder Oxy-C₁-C₂-alkylidenoxy ist.

9. Verbindungen der Formel II.9 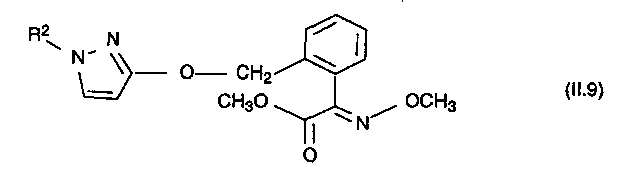 in denen R² für Phenyl steht, wobei Phenyl unsubstituiert oder substituiert mit Halogen, Cyano, C₁-C₄-Alkyl, C₁-C₄-Alkoxy, C₁-C₂-Halogenalkyl, C₁-C₂-Halogenalkoxy, Phenyl oder Oxy-C₁-C₂-alkylidenoxy ist.

10. Verbindungen der Formel II.11 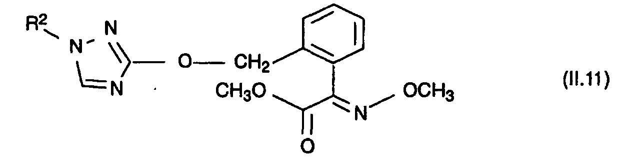 in denen R² für Phenyl steht, wobei Phenyl unsubstituiert oder substituiert mit Halogen, Cyano, C₁-C₄-Alkyl, C₁-C₄-Alkoxy, C₁-C₂-Halogenalkyl, C₁-C₂-Halogenalkoxy, Phenyl oder Oxy-C₁-C₂-alkylidenoxy ist.

11. Verwendung der Verbindungen II gemäß Anspruch 1 als Zwischenprodukte zur Herstellung durch Aminolyse von Verbindungen der Formel I 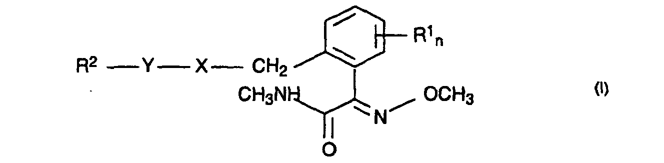 in der der Index und die Substituenten die in Anspruch 1 gegebene Bedeutung haben.

## Claims

1. A compound of the general formula II 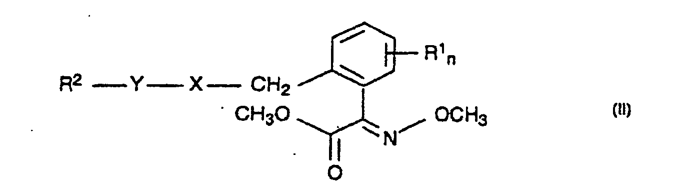 in which the index and the substituents have the following meaning:
n is 0, 1, 2, 3 or 4, where the radicals R¹ can be different if n is greater than 1;
X is O or S;
R²⁻Y is one of the following groups: 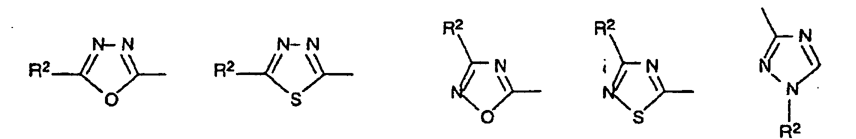 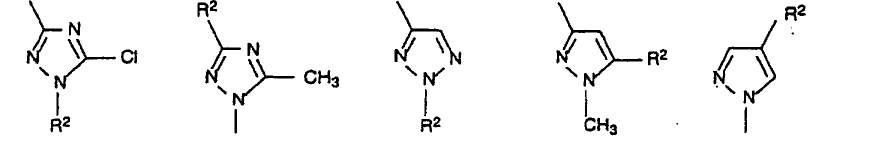 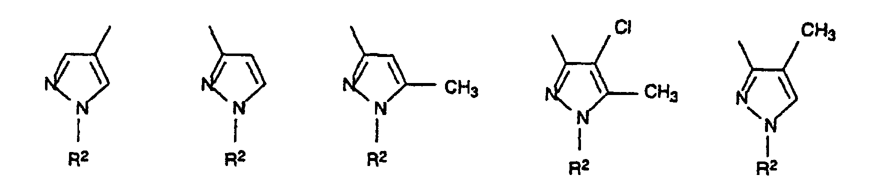 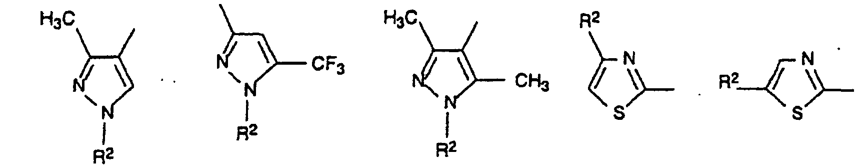 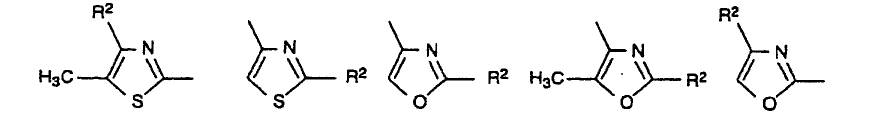 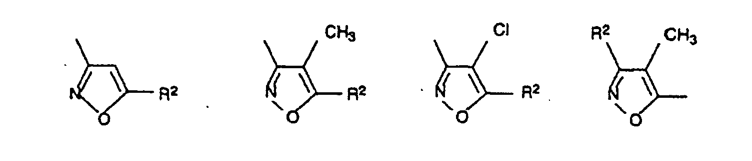
R¹ is nitro; cyano; halogen;
C₁-C₄-alkyl; C₁-C₄-haloalkyl; C₁-C₄-alkoxy; C₁-C₄-haloalkoxy; C₁-C₄-alkylthio;
phenyl or phenoxy, where the aromatic rings can carry one to five halogen atoms or one to three of the following radicals: halogen, C₁-C₄-alkoxy, C₁-C₄-haloalkyl, C₁-C₄-alkoxy, C₁-C₄-haloalkoxy and C₁-C₄-alkylthio;
or, if n is greater than 1, a 1,3-butadiene-1,4-diyl group bound to two adjacent C atoms of the phenyl radical, which for its part can carry one to four halogen atoms or one or two of the following radicals: nitro, cyano, halogen, C₁-C₄-alkyl, C₁-C₄-haloalkyl, C₁-C₄-alkoxy, C₁-C₄-haloalkoxy and C₁-C₄-alkylthio;
R² is hydrogen;
C₁-C₆-alkyl, C₂-C₆-alkenyl, or C₃-C₆-alkynyl,
where the radicals mentioned can be partially or completely halogenated and in addition to halogen atoms can additionally carry one to three of the following substituents:
nitro, cyano, thiocyanato, C₁-C₄-alkoxy, C₁-C₄-haloalkoxy, C₁-C₄-alkylthio, C₁-C₄-alkylamino, di-C₁-C₄-alkylamino, C₃-C₆-alkenyloxy, C₃-C₆-alkynyloxy, C₁-C₆-alkylcarbonyl, C₁-C₆-alkoxyimino, C₃-C₆-alkenyloxyimino, C₃-C₆-alkynyloxyimino, C₁-C₆-alkoxycarbonyl, C₁-C₆-alkylthiocarb-onyl, C₁-C₆-alkylaminocarbonyl, di-C₁-C₆ alkylaminocarbonyl, C₁-C₆-alkylcarboxyl, C₁-C₆-alkylcarbonylamino, C₃-C₇-cycloalkyl, C₃-C₇-cycloalkoxy, C₃-C₇-cycloalkylthio, C₃-C₇-cycloalkylamino, C₅-C₇-cycloalkenyl,
5- to 6-membered, saturated or unsaturated heterocycles, comprising one to three nitrogen atoms and/or an oxygen or sulfur atom,
phenyl, 1-naphthyl or 2-naphthyl,
5-membered ring heteroaromatics, comprising one to three nitrogen atoms and/or an oxygen or sulfur atom,
6-membered ring heteroaromatics, comprising one to three nitrogen atoms;
a saturated or mono- or diunsaturated ring, which in addition to carbon atoms can contain one to three of the following heteroatoms as ring members: oxygen, sulfur and nitrogen,
where the rings mentioned can be partially or completely halogenated and the mono- or diunsaturated rings, in addition to halogen atoms, can additionally carry one to three of the following substituents:
nitro, cyano, C₁-C₆-alkyl, C₁-C₄-haloalkyl, C₁-C₄-alkoxy, C₁-C₄-alkylthio, di-C₁-C₄-alkylamino, C₂-C₆-alkenyl, C₂-C₆-alkynyl, C₁-C₆-alkoxycarbonyl, C₁-C₆-alkylaminocarbonyl, di-C₁-C₆-alkylaminocarbonyl, C₁-C₆-alkylcarboxyl, C₁-C₆-alkylcarbonylamino, C₃-C₇-cycloalkyl, phenyl;
or a mono- or binuclear aromatic ring, which in addition to carbon atoms can contain one to four nitrogen atoms or one or two nitrogen atoms and an oxygen atom or sulfur atom, or an oxygen or sulfur atom as ring members,
where the rings mentioned can be partially or completely halogenated, and in addition to halogen atoms can additionally carry one to three of the following substituents:
nitro, cyano, thiocyanato, C₁-C₆-alkyl, C₂-C₆-alkenyl, C₂-C₆-alkynyl, C₃-C₆-alkynyloxy, C₁-C₆-alkylcarbonyl, di-C₁-C₆-alkylaminocarbonyl, C₁-C₆-alkylcarboxyl, C₅-C₇-cycloalkenyl,
5- to 6-membered, saturated or unsaturated heterocycles, comprising one to three nitrogen atoms and/or an oxygen or sulfur atom,
phenyl,
5-membered ring heteroaromatics comprising one to three nitrogen atoms and/or an oxygen or sulfur atom,
6-membered ring heteroaromatics comprising one to three nitrogen atoms.

2. A compound of the formula II as claimed in claim 1, in which Y is 3-pyrazolyl, 4-thiazolyl, or 1,2,4-triazol-3-yl.

3. A compound of the formula II as claimed in claim 1, in which the index and the substituents have the following meaning:
n is 0 or 1;
X is O or S;
R¹ is halogen, C₁-C₄-alkyl, C₁-C₂-haloalkyl, C₁-C₄-alkoxy, C₁-C₂-haloalkoxy, C₁-C₂-alkylthio or phenyl;
R² is phenyl, which can be unsubstituted or partially or completely halogenated and in addition to halogen atoms can additionally carry one to three of the following substituent:
nitro, cyano, thiocyanato, C₁-C₆-alkyl, C₂-C₆-alkenyl, C₃-C₆-alkynyl, C₃-C₆-alkynyloxy, C₁-C₆-alkylcarbonyl, di-C₁-C₆-alkylaminocarbonyl, C₁-C₆-alkylcarboxyl, C₅-C₇-cycloalkenyl,
5- to 6-membered, saturated or unsaturated heterocycles, comprising one to three nitrogen atoms and/or an oxygen or sulfur atom,
phenyl,
5-membered ring heteroaromatics comprising one to three nitrogen atoms and/or an oxygen or sulfur atom,
6-membered ring heteroaromatics comprising one to three nitrogen atoms.

4. A compound of the formula II as claimed in claim 1, in which the index and the substituents have the following meaning:
n is 0;
X is O;
R₁ is halogen, C₁-C₄-alkyl, C₁-C₂-haloalkyl, C₁-C₄-alkoxy, C₁-C₂-haloalkoxy, C₁-C₂-alkylthio or phenyl;
R² is phenyl, which is unsubstituted or substituted with halogen, cyano, C₁-C₄-alkyl, C₁-C₄-alkoxy, C₁-C₂-haloalkyl, C₁-C₂-haloalkoxy, phenyl or oxy-C₁-C₂-alkylideneoxy.

5. A compound of the formula II.1 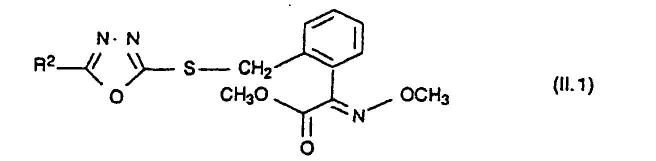 in which R² is phenyl, where phenyl is unsubstituted or substituted with halogen, cyano, C₁-C₄-alkyl, C₁-C₄-alkoxy, C₁-C₂-haloalkyl, C₁-C₂-haloalkoxy, phenyl or oxy-C₁-C₂-alkylideneoxy.

6. A compound of the formula II.2 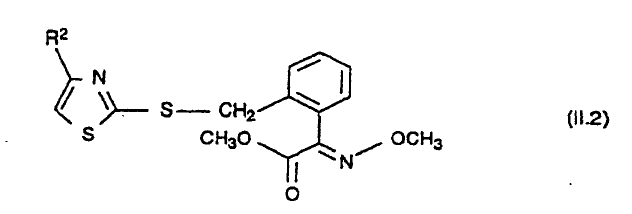 in which R² is phenyl, where phenyl is unsubstituted or substituted with halogen, cyano, C₁-C₄-alkyl, C₁-C₄-alkoxy, C₁-C₂-haloalkyl, C₁-C₂-haloalkoxy, phenyl or oxy-C₁-C₂-alkylideneoxy.

7. A compound of the formula II.3 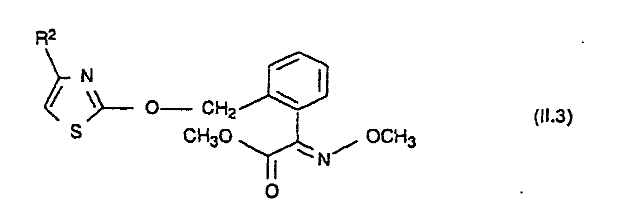 in which R² is phenyl, where phenyl is unsubstituted or substituted with halogen, cyano, C₁-C₄-alkyl, C₁-C₄-alkoxy, C₁-C₂-haloalkyl, C₁-C₂-haloalkoxy, phenyl or oxy-C₁-C₂-alkylideneoxy.

8. A compound of the formula II.5 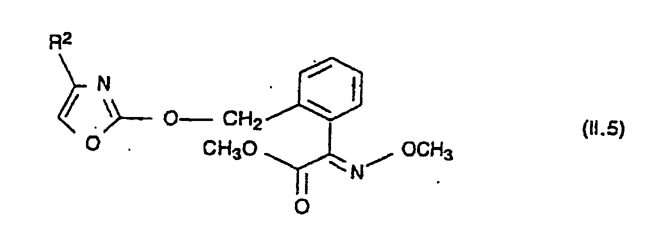 in which R² is phenyl, where phenyl is unsubstituted or substituted with halogen, cyano, C₁-C₄-alkyl, C₁-C₄-alkoxy, C₁-C₂-haloalkyl, C₁-C₂-haloalkoxy, phenyl or oxy-C₁-C₂-alkylideneoxy.

9. A compound of the formula II.9 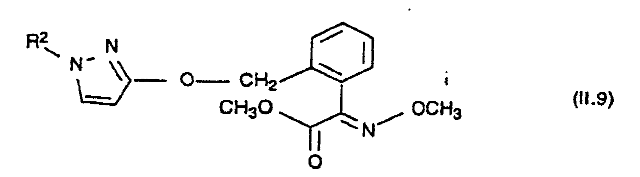 in which R² is phenyl, where phenyl is unsubstituted or substituted with halogen, cyano, C₁-C₄-alkyl, C₁-C₄-alkoxy, C₁-C₂-haloalkyl, C₁-C₂-haloalkoxy, phenyl or oxy-C₁-C₂-alkylideneoxy.

10. A compound of the formula II.11 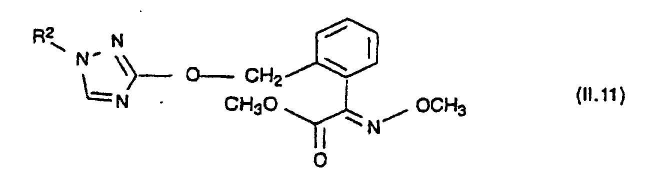 in which R² is phenyl, where phenyl is unsubstituted or substituted with halogen, cyano, C₁-C₄-alkyl, C₁-C₄-alkoxy, C₁-C₂-haloalkyl, C₁-C₂-haloalkoxy, phenyl or oxy-C₁-C₂-alkylideneoxy.

11. The use of a compound II as claimed in claim 1 as an intermediate for the preparation by aminolysis of compounds of the formula I 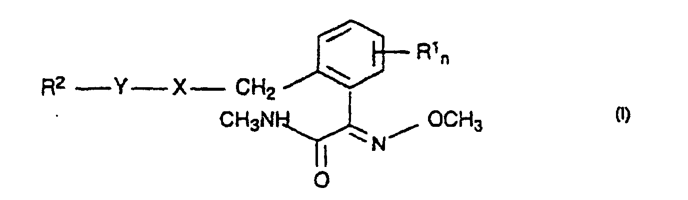 in which the index and the substituents have the meaning given in claim 1.

## Revendications

1. Composés répondant à la formule générale II 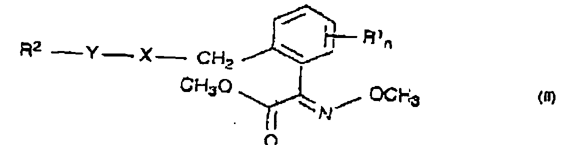 dans laquelle l'indice et les substituants ont les significations suivantes
n 0, 1, 2, 3 ou 4, les résidus R' pouvant être différents lorsque n est supérieur à 1 ;
X O ou S ;
R²-Y représente un des groupes suivants : 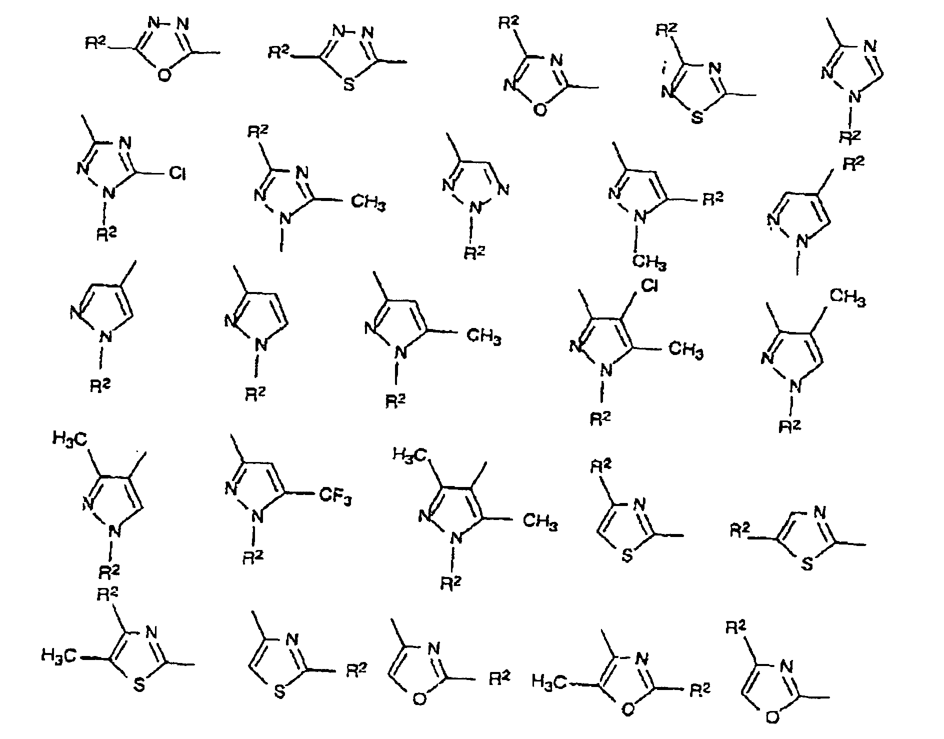 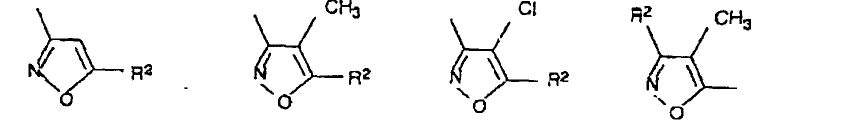
R¹ un groupe nitro, un groupe cyano, un atome d'halogène ;
un groupe alkyle en C₁-C₄, un groupe halogénalkyle en C₁-C₄, un groupe alcoxy en C₁-C₄, un groupe halogénalcoxy en C₁-C₄, un groupe (alkyle en C₁-C₄)thio ;
un groupe phényle ou un groupe phénoxy, les noyaux aromatiques pouvant porter un à cinq atomes d'halogène ou un à trois des résidus suivants : un atome d'halogène, un groupe alkyle en C₁-C₄, un groupe halogénalkyle en C₁-C₄, un groupe atcoxy en C₁-C₄, un groupe halogénalcoxy en C₁-C₄ et un groupe (alkyle en C₁-C₄)thio ;
ou, dans la mesure où n est supérieur à 1, un groupe 1,3-butadiène-1,4-diyle lié à deux atomes de carbone voisins du résidu phényle, qui peut lui-même porter un à quatre atomes d'halogènes ou 1 ou 2 des résidus suivants : un groupe nitro, un groupe cyano ; un atome d'halogène, un groupe alkyle en C₁-C₄, un groupe halogénalkyle en C₁-C₄, un groupe alcoxy en C₁-C₄, un groupe halogénalcoxy en C₁-C₄, et un groupe (alkyle
en C₁-C₄)thio ;
R² un atome d'hydrogène ;
un groupe alkyle en C₁-C₆, un groupe alcényle en C₂-C₆, ou un groupe alcinyle en C₃-C₆,
les résidus cités pouvant être partiellement ou entièrement halogénés et pouvant porter, en plus des atomes d'halogènes, un à trois des substituants suivants :
un groupe nitro, un groupe cyano, un groupe thiocyanato, un groupe alcoxy en C₁-C₄, un groupe halogénalcoxy en C₁-C₄, un groupe (alkyle en C₁-C₄)thio, un groupe (alkyle en C₁-C₄)amino, un groupe di(alkyle en C₁-C₄)amino, un groupe alcényloxy en C₃-C₆, un groupe alcinyloxy en C₃-C₆, un groupe (alkyle en C₁-C₆)carbonyle, un groupe (alcoxy en C₁-C₆)imino, un groupe (alcényle en C₃-C₆)oxyimino, un groupe (alcinyle en C₃-C₆)oxyimino, un groupe (alcoxy en C₁-C₆)carbonyle, un groupe (alkyle en C₁-C₆)thiocarbonyle, un groupe (alkyle en C₁-C₆)aminocarbonyle, un groupe di(alkyle en C₁-C₆)aminocarbonyle, un groupe (alkyle en C₁-C₆)carboxyle, un groupe (alkyle en C₁-C₆)carbonylamino, un groupe cycloalkyle en C₃-C₇, un groupe cycloalcoxy en C₃-C₇, un groupe (cycloalkyle en C₃-C₇)thio, un groupe (cycloalkyle en C₃-C₇)amino, un groupe cycloalcényle en C₅-C₇ ; hétérocycles à 5 à 6 membres, saturés ou insaturés, contenant un à trois atomes d'azote et/ou un atome d'oxygène ou de soufre,
un groupe phényle, un groupe 1-naphtyle ou un groupe 2-naphthyle, des substances hétéroaromatiques pentagonaux, contenant un à trois atomes d'azote et/ou un atome d'oxygène ou de soufre ;
des substances hétéroaromatiques hexagonaux, contenant un à trois atomes d'azote ;
un cycle saturé ou une ou deux fois insaturé, qui peut contenir, en plus des atomes de carbone, un à trois des hétéroatomes suivants comme membres du cycle : oxygène, soufre et azote,
les cycles cités pouvant être halogénés partiellement ou entièrement et les cycles une ou deux fois insaturés pouvant porter, en plus des atomes d'halogènes, un à trois des substituants suivants
un groupe nitro, un groupe cyano, un groupe alkyle en C₁-C₆, un groupe halogénalkyle en C₁-C₄, un groupe alcoxy en C₁-C₄, un groupe (alkyle en C₁-C₄)thio, un groupe di(alkyle en C₁-C₄)amino, un groupe alcényle en C₂-C₆, un groupe alcinyle en C₂-C₆, un groupe (alcoxy en C₁-C₆)carbonyle, un groupe (alkyle en C₁-C₆)aminocarbonyle, un groupe di(alkyle en C₁-C₆)aminocarbonyle, un groupe (alkyle en C₁-C₆)carboxyle, un groupe (alkyle en C₁-C₆)carbonylamino, un groupe cycloalkyle en C₃-C₇, un groupe phényle ;
ou un cycle aromatique à un ou deux noyaux, qui peut contenir, en plus des atomes de carbone, un à quatre atomes d'azote ou un ou deux atomes d'azote et un atome d'oxygène ou un atome de soufre, ou un atome d'oxygène ou un atome de soufre comme membres du cycle,
les cycles cités pouvant être partiellement ou entièrement halogénés, et pouvant porter, en plus des atomes d'halogènes, un à trois des substituants suivants :
un groupe nitro, un groupe cyano, un groupe thiocyanato, un groupe alkyle en C₁-C₆, un groupe alcényle en C₂-C₆, un groupe alcinyle en C₂-C₆, un groupe (alcinyle en C₃-C₆)oxy, un groupe (alkyle en C₁-C₆)carbonyle, un groupe di(alkyle en C₁-C₆)aminocarbonyle, un groupe (alkyle en C₁-C₆)carboxyle, un groupe cycloalcényle en C₅-C₇, hétérocycles à 5 à 6 membres, saturés ou insaturés, contenant un à trois atomes d'azote et /ou un atome d'oxygène ou un atome de soufre,
un groupe phényle, des substances hétéroaromatiques pentagonaux contenant un à trois atomes d'azote et/ou un atome d'oxygène ou un atome de soufre,
des substances hétéroaromatiques hexagonaux contenant un à trois atomes d'azote.

2. Composés répondant à la formule II selon la revendication 1, dans laquelle Y représente un groupe 3-pyrazolyle, un groupe 4-thiazolyle ou un groupe 1,2,4-triazol-3-yle,

3. Composés répondant à la formule II selon la revendication 1, dans laquelle l'indice et les substituants ont les significations suivantes :
n = 0 ou 1 ;
X = O ou S ;
R¹ = un atome d'halogène, un groupe alkyle en C₁-C₄, un groupe halogénalkyle en C₁-C₂, un groupe alcoxy en C₁-C₄, un groupe halogénalcoxy en C₁-C₂, un groupe (alkyle en C₁-C₂)thio ou un groupe phényle ;
R² = phényle, qui peut être non substitué ou partiellement ou entièrement halogéné et peut porter, en plus des atomes d'halogène, un à trois des substituants suivants :
un groupe nitro, un groupe cyano, un groupe thiocyanato, un groupe alkyle en C₁-C₆, un groupe alcényle en C₂-C₆, un groupe alcinyle en C₃-C₆, un groupe (alcinyle en C₃-C₆)oxy, un groupe (alkyle en C₁-C₆)carbonyle, un groupe di(alkyle en C₁-C₆)aminocarbonyle, un groupe (alkyle en C₁-C₆)carboxyle, un groupe cycloalcényle en C₅-C₇ ;
hétérocycles à 5 à 6 membres, saturés ou insaturés, contenant un à trois atomes d'azote et/ou un atome d'oxygène ou un atome de soufre,
un groupe phényle,
des substances hétéroaromatiques pentagonaux contenant un à trois atomes d'azote et/ou un atome d'oxygène ou un atome de soufre ;
des substances hétéroaromatiques hexagonaux contenant un à trois atomes d'azote.

4. Composés répondant à la formule II selon la revendication 1, dans laquelle l'indice et les substituants ont les significations suivantes :
n= 0 ;
X= O ;
R¹= un atome d'halogène, un groupe alkyle en C₁-C₄, un groupe halogénalkyle en C₁-C₂, un groupe alcoxy en C₁-C₄, un groupe halogénalcoxy en C₁-C₂, un groupe (alkyle en C₁-C₂)thio, ou un groupe phényle ;
R² = phényle, non substitué ou substitué par un atome d'halogène, un groupe cyano, un groupe alkyle en C₁-C₄, un groupe alcoxy en C₁-C₄, un groupe halogénalkyle en C₁-C₂, un groupe halogénalcoxy en C₁-C₂, un groupe phényle ou oxy-alkylydèneoxy en C₁-C₂.

5. Composés répondant à la formule II. 1 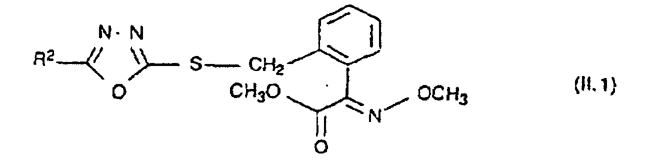 dans laquelle R² représente un groupe phényle, un groupe phényle étant non substitué ou substitué par un atome d'halogène, un groupe cyano, un groupe alkyle en C₁-C₄, un groupe alcoxy en C₁-C₄, un groupe halogénalkyle en C₁-C₂, un groupe halogénalcoxy en C₁-C₂, un groupe phényle ou un groupe oxy-alkylidèneoxy en C₁-C₂.

6. Composés répondant à la formule II.2 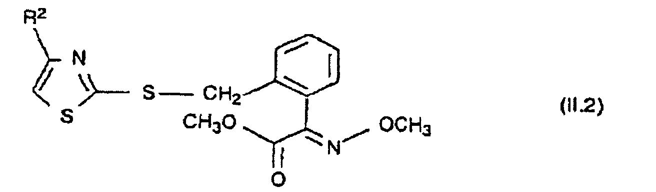 dans laquelle R² représente un groupe phényle, le groupe phényle étant non substitué ou substitué par un atome d'halogène, un groupe cyano, un groupe alkyle en C₁-C₄, un groupe alcoxy en C₁-C₄, un groupe halogénalkyle en C₁-C₂, un groupe halogénalcoxy en C₁-C₂, un groupe phényle ou un groupe oxy-alkylidèneoxy en C₁-C₂.

7. Composés répondant à la formule II.3 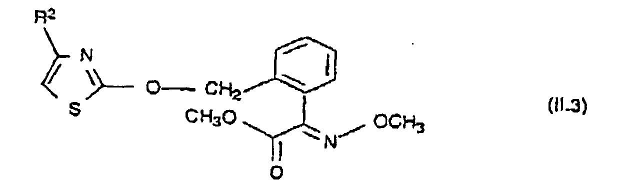 dans laquelle R² représente un groupe phényle, le groupe phényle étant non substitué ou substitué par un atome d'halogène, un groupe cyano, un groupe alkyle en C₁-C₄, un groupe alcoxy en C₁-C₄, un groupe halogénalkyle en C₁-C₂, un groupe halogénalcoxy en C₁-C₂, un groupe phényle ou un groupe oxy-alkylidèneoxy en C₁-C₂.

8. Composés répondant à la formule II.5 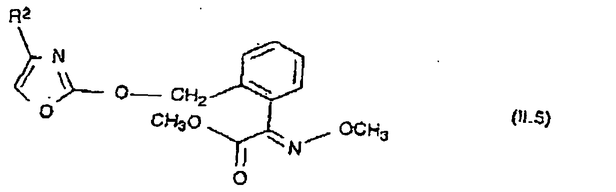 dans laquelle R² représente un groupe phényle, le groupe phényle étant non substitué ou substitué par un atome d'halogène, un groupe cyano, un groupe alkyle en C₁-C₄, un groupe alcoxy en C₁-C₄, un groupe halogénalkyle en C₁-C₂, un groupe halogénalcoxy en C₁-C₂, un groupe phényle ou un groupe oxy-alkylidèneoxy en C₁-C₂.

9. Composés répondant à la formule II.9 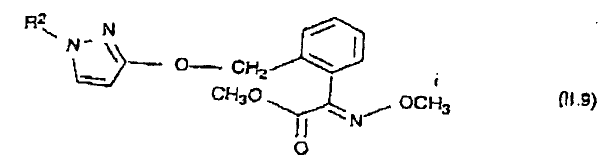 dans laquelle R² représente un groupe phényle, le groupe phényle étant non substitué ou substitué par un atome d'halogène, un groupe cyano, un groupe alkyle en C₁-C₄, un groupe alcoxy en C₁-C₄, un groupe halogénalkyle en C₁-C₂, un groupe halogénalcoxy en C₁-C₂, un groupe phényle ou un groupe oxy-alkylidèneoxy en C₁-C₂.

10. Composés répondant à la formule II. 11 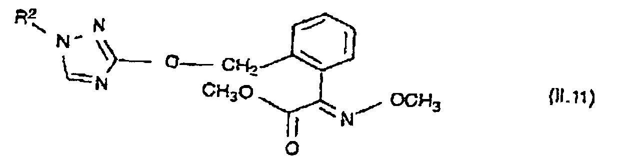 dans laquelle R² représente un groupe phényle, le groupe phényle étant non substitué ou substitué par un atome d'halogène, un groupe cyano, un groupe alkyle en C₁-C₄, un groupe alcoxy en C₁-C₄, un groupe nalogénalkyle en C₁-C₂, un groupe halogénalcoxy en C₁-C₂, un groupe phényle ou un groupe oxy-alkylidèneoxy en C₁-C₂.

11. Utilisation des composés répondant à la formule II selon la revendication 1, comme produits intermédiaires pour la préparation par aminolyse de composés répondant à la formule I, 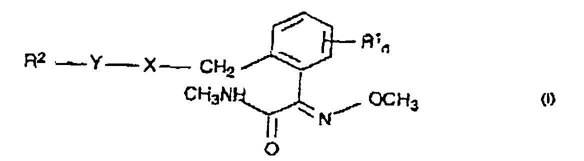 dans laquelle l'indice et les substituants ont les significations indiquées dans la revendication 1.
